# EUROPEAN PATENT APPLICATION

(11) **EP 2 860 527 A1**
(43) Date of publication of application: **15.04.2015**
(21) Application number: 13801017.8
(22) Date of filing: 06.06.2013
(51) Int. Cl.: G01N 33/68, G01N 33/53

(54) **MARKER FOR ACUTE CORONARY SYNDROME, AND USE THEREOF**

(30) Priority: 06.06.2012 JP 2012129272
(71) Applicant: Riken, Wako-shi, Saitama 351-0198 (JP)
(72) Inventor: KITAZUME, Shinobu, Wako-shi Saitama 351-0198 (JP); YOSHIHISA, Akiomi, Fukushima-shi Fukushima 960-1295 (JP); YAMAKI, Takayoshi, Fukushima-shi Fukushima 960-1295 (JP); TAKEISHI, Yasuchika, Fukushima-shi Fukushima 960-1295 (JP); HASHIMOTO, Yasuhiro, Fukushima-shi Fukushima 960-1295 (JP); TANIGUCHI, Naoyuki, Wako-shi Saitama 351-0198 (JP); IMAMAKI, Rie, Wako-shi Saitama 351-0198 (JP); YAMAMOTO, Naomasa, Saitama-shi Saitama 330-0842 (JP); SEITO, Tsutomu, Fujioka-shi Gunma 375-0005 (JP)
(74) Representative: Duckworth, Timothy John
(86) International application number: PCT/JP2013/065727
(87) International publication number: WO 2013/183720

(57) **Abstract**

The present invention provides a method of testing plasma and/or serum of a test subject for determining the pathology or onset risk of acute coronary syndrome, including
(1) a step of measuring a soluble amyloid β precursor protein (sAPP) 770 level of plasma and/or serum of a test subject; and
(2) a step of relating the plasma and/or serum sAPP770 level(s) measured in (1) to the pathology or onset risk of acute coronary syndrome.

## Description

### Technical Field

The present invention relates to a marker for acute coronary syndrome and use thereof. More particularly, the present invention relates to a method of testing plasma and/or serum of a test subject for the determination of the pathology of acute coronary syndrome, a diagnostic agent to be used therefor and the like.

### Background Art

Deposition of amyloid β(Aβ) in the brain is closely related to Alzheimer's disease (AD) (non-patent document 1). Aβ is produced from an amyloid precursor protein (APP), which is a single-pass transmembrane protein, by sequential cleavage by β-secretase (β-site amyloid precursor protein cleaving enzyme; BACE1) and γ-secretase, and extracellularly released (non-patent document 2). In addition, APP can be cleaved at an α site in the Aβ sequence by α-secretase. As a result of the cleavage of APP at α site and β site, the N-terminal regions of APP, which are called sAPPα and sAPPβ, respectively, are extracellularly released. Furthermore, APP contains three kinds of isoforms APP695, APP751 and APP770 due to selective splicing (non-patent documents 3 and 4), and APP695 is mainly expressed in neuron (non-patent document 5). Many evidence suggest that AD patients initially show a cerebrovascular brain lesion (non-patent documents 6-11). It is also an important finding that Aβ deposition in the arteriolar wall of the brain occurs in almost all AD patients (called cerebrovascular amyloid angiopathy) (non-patent documents 12-15). The present inventors have already found that vascular endothelial cells express APP770 and produce Aβ40/42 (non-patent document 16). While the derivation of Aβ that deposits on the blood vessel is controversial, a recent study of Aβ immunotherapy has revealed that Aβ deposition in cerebral parenchyma and blood vessel occurs independently (non-patent document 17).

In connection with Alzheimer's disease, production of sAPPα and sAPPβ as well as α cleavage activity and β cleavage activity have heretofore been analyzed in depth (non-patent documents 18 and 19).

Recently, the present inventors invented a sandwich ELISA system that specifically detects soluble APP770P (sAPP770β), which is an N-terminal fragment of BACE1 cleavage product of APP770 (patent document 1). Using this system, they have found that sAPP770β of vascular endothelial cell can potentially become a biomarker of diseases accompanied by Aβ accumulation, such as cerebrovascular amyloid angiopathy and Alzheimer's disease.

In the meantime, apart from dementia such as Alzheimer's disease and the like, acute coronary syndrome is also one of the important diseases having a large social impact. The number of angina pectoris patients is estimated to be about 650,000 in 2010. In actual clinical situation, it is important to distinguish acute coronary syndromes (unstable angina pectoris, acute myocardial infarction), for which an invasive treatment (urgent cardiac catheter test) is urgently necessary, from stable angina pectoris. Representative acute coronary syndrome markers (troponin T, troponin I, H-FABP, AST, CPK, LDH and the like), which have currently been established, show an increase only when a disorder in the cardiac muscle (myocardial damage), rather than a vascular disorder, has occurred. On the other hand, a marker that shows property and instability of plaque in blood vessels has not been established.

### [Document List]

### [patent document]

patent document 1: WO 2012/015050

### [non-patent documents]

non-patent document 1: Physiol Rev 81(2): 741-766 (2001)
non-patent document 2: Physiol Rev 90(2):65-494 (2010)
non-patent document 3: Nature 331(6156):525-527 (1988)
non-patent document 4: Nature 331(6156):528-530 (1988)
non-patent document 5: Proc Natl Acad Sci USA 90(20):9513-9517 (1993)
non-patent document 6: Neurology 38(6):931-937 (1988)
non-patent document 7: JAMA 277(10):813-817 (1997)
non-patent document 8: Neurology 51(4):1009-1013 (1998)
non-patent document 9: Neurobiol Aging 21(2):153-160 (2000)
non-patent document 10: N Engl J Med 348(13):1215-1222 (2003)
non-patent document 11: Journal of Neuroscience 28(50):13542-13550 (2008)
non-patent document 12: Neurology 25(2):120-126 (1975)
non-patent document 13: Ann Pathol 1(2):120-129 (1981)
non-patent document 14: Stroke 18(2):311-324 (1987)
non-patent document 15: Lancet 354(9182):919-920 (1999)
non-patent document 16: J Biol Chem 285(51):40097-40103 (2010)
non-patent document 17: Nature Medicine 9(4):448-452 (2003)
non-patent document 18: Journal of Neuroscience 28(46):12052-12061 (2008)
non-patent document 19: J Neurosci Res 89(6):822-832 (2011)

### SUMMARY OF THE INVENTION

### Problems to be Solved by the Invention

The present invention aims to further elucidate the role of sAPP770, which is a soluble form of a selective splicing variant of APP, in the living body, clarify the relationship with diseases other than dementia such as Alzheimer's disease and the like, and establish a biomarker of the diseases.

### Means of Solving the Problems

The present inventors have conducted intensive studies in an attempt to solve the above-mentioned problems, and unexpectedly found that sAPP770, which is secreted from vascular endothelial cells by inflammatory cytokines, increases, and sAPP770 is degranulated from activated platelets and released in large amounts. Blood vessel endothelial damage, platelet activation and thrombus formation are important phenomena in the development and progression of atherosclerosis, and the development of acute coronary syndrome (ACS) (N Engl J Med 357(24):2482-2494 (2007)). Therefore, the level of sAPP770 in the biological samples derived from ACS patients (including unstable angina pectoris patients and acute myocardial infarction (AMI) patients) was measured. As a result, they have found that the plasma sAPP770 level is significantly higher in AMI patients than healthy human, the serum sAPP770 level is significantly low in AMI patients, and that as the pathology of acute coronary syndrome progresses more, the plasma sAPP770 level becomes higher, which resulted in the completion of the present invention.

More specifically, the present invention provides the following.
[1] A method of testing plasma and/or serum of a test subject for determining the pathology or onset risk of acute coronary syndrome, comprising
   (1) a step of measuring a soluble amyloid β precursor protein (sAPP) 770 level of plasma and/or serum of a test subject; and
   (2) a step of relating the plasma and/or serum sAPP770 level(s) measured in (1) to the pathology or onset risk of acute coronary syndrome.
[2] The method of [1], wherein the step (1) comprises measuring the plasma sAPP770 level of the test subject and the step (2) comprises relating the plasma sAPP770 level to the pathology or onset risk of acute coronary syndrome.
[3] The method of [1], wherein the step (1) comprises measuring the plasma and serum sAPP770 levels of the test subject and the step (2) comprises relating the ratio of plasma sAPP770 level/serum sAPP770 level to the pathology or onset risk of acute coronary syndrome.
[4] The method of [1], wherein the step (1) comprises measuring the serum sAPP770 level of the test subject, and the step (2) comprises relating the serum sAPP770 level to the pathology or onset risk of acute coronary syndrome.
[5] The method of any of [1] - [4], wherein the step (2) comprises relating the plasma and/or serum sAPP770 level(s) measured in step (1) to the pathology of acute coronary syndrome.
[6] The method of [5], which is a test method of plasma and/or serum of a test subject for determining severity of acute coronary syndrome.
[7] The method of [5], which is a test method of plasma and/or serum of a test subject for distinguishing acute coronary syndrome from non-acute coronary syndrome.
[8] The method of [5], which is a test method of plasma and/or serum of a test subject for determining whether the prognosis of acute coronary syndrome is good or bad.
[9] The method of any of [1] - [4], which is a test method of plasma and/or serum of a test subject for determining the onset risk of acute coronary syndrome, wherein the step (2) comprises relating the plasma and/or serum sAPP770 level(s) measured in the step (1) to the onset risk of acute coronary syndrome.
[10] The method of any of [1] - [9], wherein the sAPP770 level is detected using an sAPP770-specific antibody.
[11] The method of [10], wherein the sAPP770-specific antibody recognizes an OX2 domain.
[12] The method of any of [1] - [11], wherein the sAPP770 level is measured by sandwich ELISA.
[13] A diagnostic agent comprising an sAPP770-specific antibody for determining the pathology or onset risk of acute coronary syndrome based on the plasma and/or serum sAPP770 level(s) of a test subject.
[14] The agent of [13], wherein the sAPP770-specific antibody recognizes an OX2 domain.
[15] The agent of [13] or [14], further comprising an antibody recognizing sAPP, which is different from the aforementioned sAPP770-specific antibody.
[16] A diagnosis kit comprising an sAPP770-specific antibody for determining the pathology or onset risk of acute coronary syndrome based on the plasma and/or serum sAPP770 level(s) of a test subject.
[17] The kit of [16], wherein the sAPP770-specific antibody recognizes an OX2 domain.
[18] The kit of [16] or [17], further comprising an antibody recognizing sAPP, which is different from the aforementioned sAPP770-specific antibody.

### Effect of the Invention

According to the present invention, the pathology of acute coronary syndrome can be evaluated rapidly and highly accurately based on the sAPP770 levels of plasma and/or serum. The basic pathology of acute coronary syndrome includes coronary plaque rupture and platelet activation. In the present invention, a phenomenon reflecting a disorder of blood vessel endothelium is detected rather than a disorder of cardiac muscle, which enables grasp of the pathology of acute coronary syndrome in an earlier stage. Previous prediction of whether the onset risk of ACS is high or low and the like (e.g., whether it is an initial stage of arteriosclerosis development) is also expected to be made based on the evaluation of the blood vessel hypofunction and platelet activity of patients with lifestyle-related diseases (hypertension patients, diabetes patients, hyperlipemia patients, sleep apnea syndrome patients, smokers) who are potential ACS patients. Furthermore, according to the present invention or according to a combination of the present invention and other test means (CT, ultrasonography etc.), ACS can be distinguished from non-ACS similar thereto (e.g., distinction of unstable angina pectoris from stable angina pectoris and the like), a treatment plan relating to the application of an invasive treatment (catheter treatment and the like) can be facilitated, and prognosis of ACS can also be determined.

### Brief Description of the Drawings

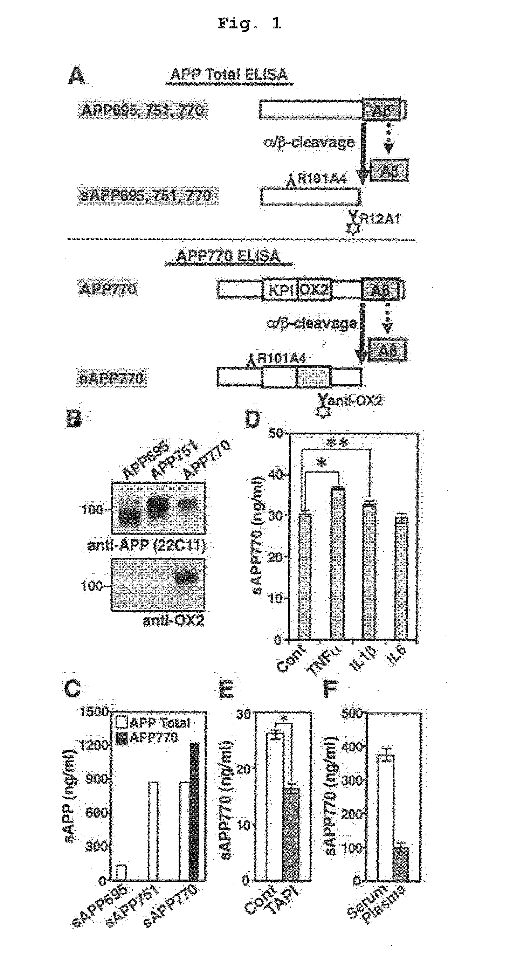

Fig. 1 shows detection of sAPP770 secreted from brain endothelial cell, sAPP770 in human serum sample and sAPP770 in human plasma sample, by using the APP770 ELISA system established by the present inventors. (A) Brief explanations of APP total ELISA and APP770 ELISA. The recognition sites of a trap antibody (with asterisk) and a detection antibody (without asterisk) in these ELISA systems are shown in the schematic drawing. (B) Western blot analysis. Anti-N terminal APP (22C11) antibody detects full-length APP695, APP751 and APP770 in the lysates of COS cells overexpressing APP695, APP751 and APP770, whereas anti-OX2 antibody used as a trap antibody in APP770 ELISA specifically recognizes APP770. (C) APP total ELISA detects sAPP695, sAPP751 and sAPP770 secreted from COS cells, whereas APP770 ELISA detects only sAPP770. (D) BMEC was cultured for 16 hr in the presence or absence of inflammatory cytokine (TNFα, IL-1β or IL-6; each 100 ng/ml), and sAPP770 secreted from the cells was detected by APP770 ELISA. The data shows mean±SEM (n=4). *P<0.00001;**P<0.05. (E) An influence on secretion of sAPP770 from BMEC by culture for 16 hr in the presence of TAPI-0 (10 µM). The data shows mean±SEM (n=4). *P<0.0005. (F) The sAPP770 levels of human serum and human plasma samples are shown in mean±SEM (n=19).

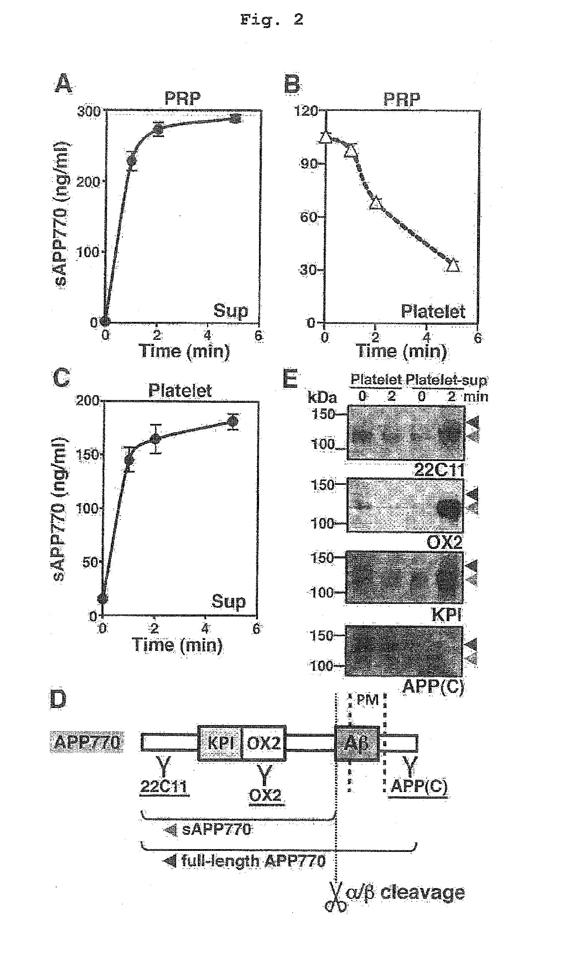

Fig. 2 shows release of sAPP770 from activated platelets. (A, B) PRP was stirred under stimulation with collagen (final concentration 3 µg/ml). At the time points shown in the Figure, each sample was centrifuged, and supernatant (A) solubilized with T-PER buffer and platelet pellet (B) were analyzed by APP770 ELISA. (C) Platelets were washed, stimulated with collagen, centrifuged, and sAPP770 released in the supernatant was analyzed. (D) Schematic drawing showing APP770 and the sites recognized by a series of anti-APP antibodies. (E) After stimulation of platelets with collagen and, at the time points shown in the Figure, the platelet pellets were solubilized, sAPP was pulled down from the platelet release product, and analyzed by western blotting using anti-APP(22C11) antibody, anti-OX2 antibody, anti-APP(C) antibody and anti-KPI antibody. Black and gray arrow heads show full-length APP770 and sAPP770, respectively.

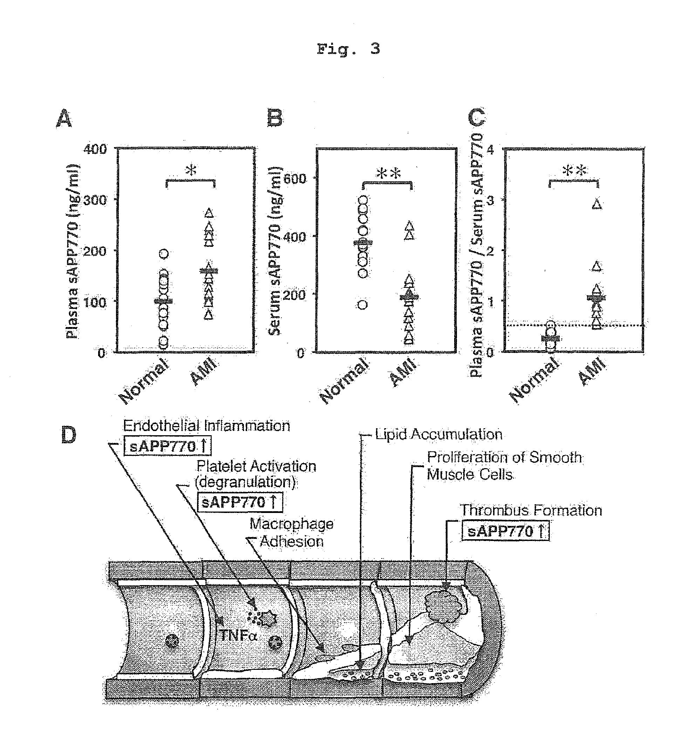

Fig. 3 shows that plasma sAPP770 is significantly higher and serum sAPP770 is significantly lower in AMI patients than in healthy human. (A, B) Plasma sAPP770 (A) and serum sAPP770 (B) derived from peripheral blood samples of healthy human and AMI patients were measured. The horizontal line shows average of each group. *P<0.005;**P<1x10⁻⁵. (C) The ratio of plasma sAPP770 to serum sAPP770 in AMI patients and normal case. The horizontal line shows average of each group. **P<1x10⁻⁵. (D) Pathology cascade of ACS. Plasma sAPP770 is considered to increase due to endothelial inflammation, platelet activation, and thrombus formation.

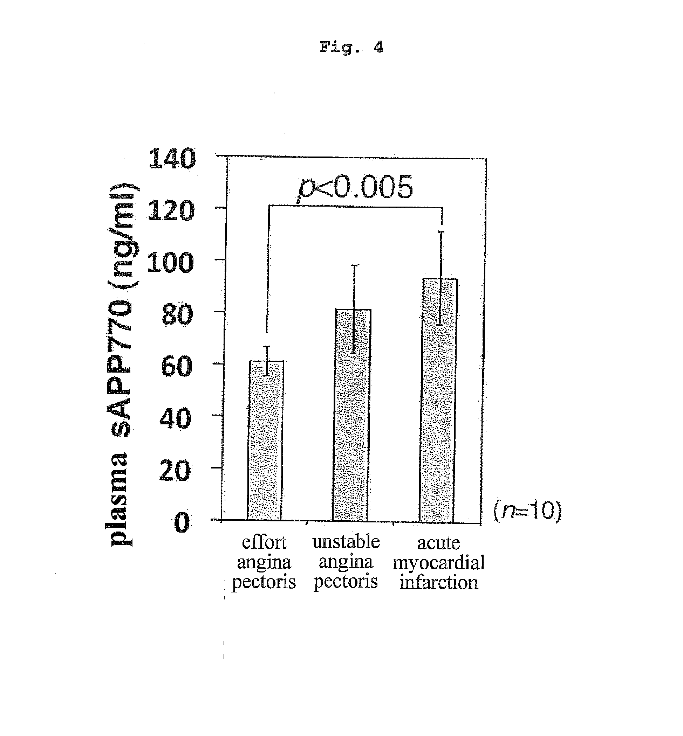

Fig. 4 shows that the plasma sAPP770 level is higher in acute coronary syndrome patients (unstable angina pectoris patients and acute myocardial infarction patients) than in effort angina pectoris patients. The data shows mean±SEM (n=10).

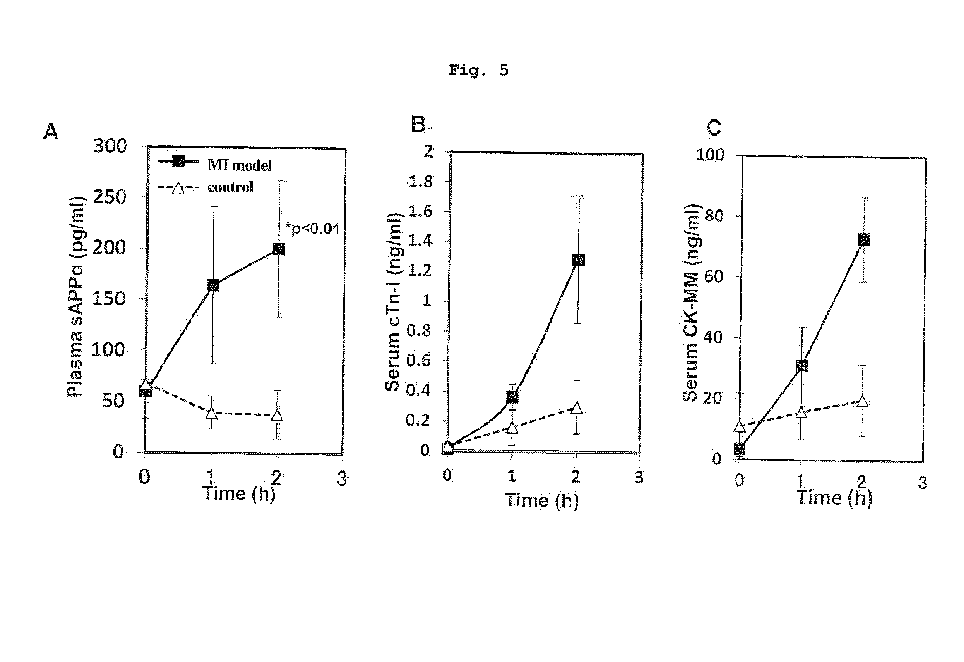

Fig. 5 shows that, in myocardial infarction (MI) model rat, sAPPα(A) increases prior to cardiac muscle troponin I (cTn-I) (B) and creatine kinase (CK-MM) (C), which are myocardial infarction markers. The data shows mean±SEM (n=8).

### Description of Embodiments

### Definition

In the present invention, amyloid β precursor protein (APP) means a molecule that forms, when cleaved by α-secretase (α cleavage), soluble amyloid by cleavage by γ-secretase (γ cleavage) thereafter or, when cleaved by β secretase (β cleavage), amyloid β peptide by γ cleavage thereafter. As one example, 3 kinds of splicing variants of APP695, APP751 and APP770 are known for human APP.

The amino acid sequence of APP and a base sequence encoding same have been made public for several kinds of animals. As for human, the amino acid sequences (GenBank Accession Nos.NP_958817, NP_958816 and NP_000475, respectively) and the base sequences (GenBank Accession Nos.NM_201414.1, NM_201413.1 and NM_000484.2, respectively) of 3 kinds of APP splicing variants of APP695, APP751 and APP770 have been made public, and can be isolated by a method known per se. The information of amino acid sequence and base sequence of APP derived from an animal other than human can also be obtained easily by searching the genome databases of various animals, and can be isolated by a method known per se based on the information. The following explanation is based on human APP as an example.

As compared to APP695, APP751 further contains a Kunitz-type protease inhibitor (KPI) region, and APP770 contains OX2 region in addition to the KPI region. These 3 splicing variants have the same amino acid sequence in a region other than KPI region and OX2 region (including C-terminal side region containing Aβ region and transmembrane region). Therefore, the OX2 region is a characteristic sequence usable for distinguishing APP770 from the other two variants.

In the present invention, soluble amyloid β precursor protein (sAPP) means an N-terminal side fragment of APP produced by cleavage by α secretase (called α cleavage) or β secretase (called β cleavage), which does not include the C-terminal side fragment. The cleavage products produced from APP splicing variants of APP695, APP751 and APP770 are referred to as sAPP695, sAPP751 and sAPP770, respectively.

The soluble amyloid β precursor protein (sAPP) 770 level in the present invention shows the content of sAPP770 in a given amount of a sample. The sAPP770 level can be shown in a manner known to those of ordinary skill in the art, according to the measurement method to be mentioned later. For example, it may be shown as a concentration of sAPP770, or as a relative value based on the measured value of a standard sample.

The sAPP770 level in the present invention is generally a total of α cleavage product and β cleavage product. As the APP cleavage products in the living body, the amount of α cleavage product is generally larger than that of β cleavage product (Selkoe, D.J., Cell biology of protein misfolding: the examples of Alzheimer's and Parkinson's diseases. Nat. Cell Biol., 6, 1054-1061 (2004); Etcheberrigaray, R., et al., Therapeutic effects of PKC activators in Alzheimer's disease transgenic mice. Proc. Natl. Acad. Sci. USA, 101, 11141- 11146 (2004)). Therefore, the sAPP770 level in the present invention may be of the level of α cleavage product.

The antibody in the present invention comprises, but is not limited to, all classes and subclasses of immunoglobulin and also comprises forms of functional fragment of antibody, and the said antibody comprises natural antibodies and also comprises antibodies, antibody fragments, and binding fragments of these, which can be produced by gene recombination technique. The said antibody also comprises polyclonal antibodies which are antibody preparations containing different antibodies against different epitopes, and monoclonal antibodies which are antibodies (including antibody fragments) obtained from substantially uniform antibody population. A binding fragment means a partial region of one of the above-described antibodies; specifically including, for example, F(ab')₂, Fab', Fab, Fv (variable fragment of antibody), sFv, dsFv (disulphide stabilized Fv), dAb (single domain antibody) and the like (Exp. Opin. Ther. Patents, Vol.6, No.5, p.441-456, 1996).

In the present invention, acute coronary syndrome is a pathology caused by acute coronary obstruction, and is a concept of diseases from unstable angina pectoris (case that did not develop cardiac muscle necrosis) to acute myocardial infarction (case that developed cardiac muscle necrosis). Therefore, the "acute coronary syndrome" in the present invention intends to include unstable angina pectoris and acute myocardial infarction.

In the present invention, moreover, non-acute coronary syndrome is a pathology wherein symptoms (anterior chest pain, tight chest feeling etc.) similar to those of acute coronary syndrome are developed but before acute coronary syndrome. For example, stable angina pectoris, effort angina pectoris, non-cardiac chest pain and the like are included.

To determine the pathology or onset risk in the present invention intends to include evaluation and testing in accordance with a certain standard.

### 1. Test method of plasma and/or serum of test subject for determination of pathology or onset risk of acute coronary syndrome

The present invention provides a method of testing plasma and/or serum of a test subject for determining the pathology or onset risk of acute coronary syndrome, comprising
(1) a step of measuring a soluble amyloid β precursor protein (sAPP) 770 level of plasma and/or serum of a test subject; and
(2) a step of relating the plasma and/or serum sAPP770 level(s) measured in (1) to the pathology or onset risk of acute coronary syndrome (hereinafter to be also referred to as the test method of the present invention).

To determine the pathology of acute coronary syndrome in the present invention means to determine the stage of acute coronary syndrome the test subject is in, and includes not only determination of the severity of acute coronary syndrome of the test subject, but also determination of whether the test subject has developed acute coronary syndrome (particularly acute myocardial infarction), distinguishing acute coronary syndrome from symptoms similar thereto (e.g., effort angina pectoris etc.), and determination of whether the prognosis of acute coronary syndrome is good or bad. The test method of the present invention is based on the finding, for the first time, of the correlation between the plasma and/or serum sAPP770 level(s) of patients with acute coronary syndrome (including patients with unstable angina pectoris and patients with acute myocardial infarction), and the pathology of acute coronary syndrome. According to the present invention, the pathology of acute coronary syndrome can be determined rapidly and highly accurately by examining the plasma and/serum of the test subject.

In the present invention, moreover, to determine the onset risk of acute coronary syndrome means previous determination of whether or not a test subject has a risk of developing acute coronary syndrome, and includes determination of whether or not a test subject (particularly patients with lifestyle-related diseases) has a risk of developing acute coronary syndrome, and determination of whether the onset risk is high or low. Myocardial infarction markers (troponin T, troponin I, H-FABP, AST, CPK, LDH etc.) used for conventional blood biochemical tests show a disorder of cardiac muscle and increase only when a blood flow disorder develops into a cardiac muscle disorder. On the other hand, the plasma and/or serum sAPP770 level(s) is(are) considered to reflect the inflammation state of vascular endothelial cells known to be related to a comparatively initial stage of pathological cascade that leads to acute coronary syndrome, activation state of platelets and the like, and increase(s) in an earlier stage as compared to conventional markers. Therefore, the present invention can determine the onset risk before the test subject develops acute coronary syndrome and can also identify, for example, potential acute coronary syndrome patients (e.g., patients with lifestyle-related diseases and in the initial stage of developing arteriosclerosis).

The test subject in the present invention may be any mammal, preferably a mammal affected with or suspected to be affected with acute coronary syndrome. Examples of the mammal include rodents such as mouse, rat, hamster, guinea pig and the like, experiment animals such as rabbit and the like, pets such as dog, cat and the like, domestic animals such as bovine, swine, goat, horse, sheep and the like, primates such as monkey, orangutan, chimpanzee and the like, and human and the like, with particular preference given to human. The test subject may or may not show the pathology of acute coronary syndrome, and may or may not be under treatment for acute coronary syndrome.

The plasma and serum of a test subject can be prepared by a method known per se from a peripheral blood sample collected from the test subject. The sample may be appropriately diluted with a known buffer and the like according to the means for sAPP770 measurement. For example, it may be diluted 75- to 100-fold or more with a dilution buffer attached to a human sAPP total assay kit (IBL-Japan) and the like, and the like. When time is necessary before measurement of blood samples after collection thereof, the plasma and/or serum can be cryopreserved up to the measurement.

The sAPP770 level in step (1) can be measured by an immunological method using an antibody that specifically recognizes sAPP770 (i.e., sAPP770-specific antibody). Examples of the immunological method include antibody array, flow cytometry analysis, radio immunoassay (RIA method), ELISA (Methods in Enzymol. 70: 419-439 (1980)), Western blotting, immunohistostaining, enzyme immunoassay (EIA method), fluorescent immunoassay (FIA), immunochromatography method and the like. Preferred is ELISA from the aspects of sensitivity and easiness of operation.

The "specific recognition" of antigen X by an antibody means that the affinity of the antibody for antigen X is stronger than the affinity for antigen other than antigen X in an antigen-antibody reaction. In the present specification, an antibody that specifically recognizes antigen X is sometimes abbreviated as "anti-X antibody" or "X-specific antibody".

The APP770-specific antibody may be any of a polyclonal antibody and a monoclonal antibody, and may be a binding fragment of these.

The aforementioned antibodies may be directly or indirectly labeled with a labeling substance. Examples of the labeling substance include fluorescent substances (e.g., FITC, rhodamine), radioactive substances (e.g., ³²P, ³⁵S, ¹⁴C, ³H), enzymes (e.g., alkaline phosphatase, peroxidase), colored particles (e.g., colloidal metal particles, colored latex), biotin and the like.

The aforementioned antibody can also be used in a soluble state without conjugation, or may be conjugated to a solid phase. The "solid phase" includes plate (e.g., microwell plate), tube, bead (e.g., plastic bead, magnetic bead), carrier for chromatography (e.g., water-absorbing substrate such as nitrocellulose membrane, Sepharose), membrane (e.g., nitrocellulose membrane, PVDF membrane), gel (e.g., polyacrylamide gel), metal film (e.g., gold film), and the like. Of these, plate, bead, carrier for chromatography, and membrane are preferably used, and plate is most preferably used since handling is easy. Examples of the above-mentioned conjugation include, but are not particularly limited to, covalent bond, ionic bond, physical adsorption, and the like, and covalent bond and/or physical adsorption are/is preferable, since sufficient binding strength can be afforded. The conjugation to a solid phase may be a direct conjugation to a solid phase, or an indirect conjugation to a solid phase by utilizing a substance known per se. In addition, it is a general practice to contact a solid phase with a phosphate-buffered solution of bovine serum albumin (BSA), cow milk protein and the like, and block a solid phase surface, which was not coated with the antibody, with the aforementioned BSA, cow milk protein and the like to suppress non-specific adsorption and non-specific reaction.

The embodiment, order, specific method, and the like to contact sAPP770-specific antibody with plasma or serum derived from test subject are not particularly limited if it is a method that the antibody is able to interact with sAPP770 in the plasma or serum. The contact may be performed, for example, by adding protein in plasma or serum to a plate in which an antibody is solid phased. Alternatively, for example, it may be also performed by contacting antibodies with biological sample separated by a method such as SDS-PAGE, and transferred and immobilized on a membrane.

While the time of the contact to be maintained is not particularly limited as long as it is sufficient for the aforementioned antibody and sAPP770 contained in plasma or serum derived from a test subject to be bound to each other to form a complex, it is generally several seconds - over 10 hours. The temperature condition for the contact is generally 4°C - 50°C, preferably 4°C - 37°C, most preferably room temperature of about 15°C - 30°C. The pH condition of the reaction is preferably 5.0 - 9.0, particularly preferably a neutral range of 6.0 - 8.0.

In a preferable embodiment of the present invention, the measurement of sAPP770 level in step (1) can be performed by a sandwich ELISA method using an sAPP770 -specific antibody and an antibody recognizing soluble amyloid β precursor protein (sAPP) different from the antibody. Such sandwich ELISA method is suitable for practicing the present invention since it has high specificity for antigen.

As used herein, the "antibody recognizing sAPP" means an antibody with an ability to bind to any sAPP, including the above-mentioned sAPP695, sAPP751 and sAPP770, or at least sAPP770. Moreover, the "antibody recognizing sAPP" preferably has an ability to bind to at least an α cleavage product of APP. Such antibodies can be prepared, for example, by a method known per se, based on the information of amino acid sequence or the state of sugar chain addition and the like that are common in sAPPs. Such antibodies are also commercially available (for example, mouse monoclonal anti-APP 22C11 antibody (Chemicon), anti-KPI antibody (Chemicon), and the like).

The sandwich ELISA method is performed, for example, as follows. Firstly, one of the antibodies is solid phased on the well surface of an ELISA plate. After blocking to prevent non-specific adsorption onto the well surface, a sample is added to allow contact of sAPP770 in the sample with the antibody to form a complex. Protein not bound with the antibody is removed by washing, the other labeled antibody is added to the well to allow contact with sAPP770 to form a complex, and detection and quantification are performed using the label. Quantification can be performed by a method known per se, such as digitizing the signal detected based on the label used, by utilizing the analytical curve formed using a standard sample of sAPP770, and the like.

For sandwich ELISA method, a labeled antibody, for example, enzyme labeled antibody, can be used. As the enzyme usable as a label, peroxidase, alkaline phosphatase, glucose oxidase, β-galactosidase and the like can be exemplified. As the substrate used for the enzyme detection, a substrate suitable for the selected enzyme label is employed. For example, when peroxidase is selected as the enzyme, o-phenylenediamine (OPD), tetramethylbenzidine (TMB) and the like are used and, when alkaliphosphatase is selected, p-nitrophenylphosphate (PNPP) and the like are used. As the reaction stop solution and substrate solution, conventionally-known ones can be appropriately used without particular limitation, according to the selected enzyme.

Next, in step (2), the plasma and/or serum sAPP770 level(s) of the test subject measured in step (1) is related to the pathology or onset risk of acute coronary syndrome.

To relate the plasma and/or serum sAPP770 level(s) of the test subject to the pathology of acute coronary syndrome means to decide whether the data of the test subject suggests (or indicates) the pathology (e.g., unstable angina pectoris, acute myocardial infarction etc.) of acute coronary syndrome. The data of the test subject is generally related to the pathology of acute coronary syndrome by comparison of the data of the test subject and the data obtained from acute coronary syndrome patients, comparison of the data of the test subject and the data obtained from non-acute coronary syndrome patients, and comparison of the data of the test subject and the data obtained from healthy human. When the data of the test subject (plasma sAPP770 level, serum sAPP770 level, or the plasma sAPP770 level/serum sAPP770 level ratio) does not show a statistically significant difference (generally, p<0.005) from the data of patients with particular pathology (e.g., unstable angina pectoris patients, acute myocardial infarction patients etc.), the data of the test subject can be related to the pathology. In this way, the test subject can be determined to have the pathology, the severity of acute coronary syndrome of the test subject can be determined, and the prognosis of acute coronary syndrome can be determined to be good or bad.

The data of the test subject may also be related to the pathology of acute coronary syndrome by comparing the data of the test subject and the data of patients other than the test subject, who have non-acute coronary syndromes (e.g., effort angina pectoris etc.) similar to acute coronary syndrome. When the plasma sAPP770 level, or the ratio of plasma sAPP770 level and serum sAPP770 level of the test subject is higher (preferably, statistically significantly higher (generally, p<0.005)) than that of patients with non-acute coronary syndrome similar to acute coronary syndrome, the data of the test subject can be related to the pathology of acute coronary syndrome, and acute coronary syndrome can be distinguished from symptoms similar thereto. Also, when the serum sAPP770 level of the test subject is lower than that of healthy human (preferably, statistically significantly lower (generally, p<0.005)), the data of the test subject can be related to the pathology of acute coronary syndrome. In this way, the test subject can be determined to have developed acute coronary syndrome.

To relate the plasma and/or serum sAPP770 level(s) of the test subject to the onset risk of acute coronary syndrome means to decide whether the data of the test subject suggests (or indicates) the onset risk of acute coronary syndrome.

The data of the test subject is generally related to the onset risk of acute coronary syndrome by comparing the data of the test subject and the data of a healthy human other than the test subject. When the plasma sAPP770 level, or the plasma sAPP770 level/serum sAPP770 level ratio of the test subject is higher (preferably, statistically significantly (generally, p<0.005) higher than that of a healthy human), the data of the test subject can be related to the onset risk of acute coronary syndrome, and the test subject can be determined to have the onset risk of acute coronary syndrome. Furthermore, it can be determined that a greater difference between the values of the test subject and healthy human means that the onset risk of acute coronary syndrome of the test subject is higher. Also, when the serum sAPP770 level of the test subject is lower than that of healthy human (preferably, statistically significantly lower (generally, p<0.005)), the data of the test subject can be related to the onset risk of acute coronary syndrome, and the test subject can be determined to have the onset risk of acute coronary syndrome. Furthermore, it can be determined that a greater difference between the values of the test subject and healthy human means that the onset risk of acute coronary syndrome of the test subject is higher.

For the above-mentioned relating, the plasma and/or serum sAPP770 level(s) of the measured test subject may be compared to the average plasma and/or serum sAPP770 level(s) or the average plasma sAPP770 level/serum sAPP770 level ratio, which were determined in advance, of many patients who have developed acute coronary syndrome, or many patients with non-acute coronary syndrome similar to acute coronary syndrome, or many healthy humans.

As shown in the below-mentioned Examples, the plasma sAPP770 level of the acute coronary syndrome patients was higher than the plasma sAPP770 level of the effort angina pectoris patients. Among the acute coronary syndromes, the plasma sAPP770 level of patients with highly severe symptoms of acute myocardial infarction was higher than that of unstable angina pectoris patients with comparatively low severity. That is, the pathology of acute coronary syndrome can be determined by measuring the plasma sAPP770 level of the test subject and relating the level to the pathology of acute coronary syndrome. For example, when the plasma sAPP770 level of a test subject is significantly higher than that of a patient with non-acute coronary syndrome similar to acute coronary syndrome, such as effort angina pectoris and the like, the test subject can be determined to have acute coronary syndrome, and further, it can be determined that a higher plasma sAPP770 level of the test subject means that the severity of acute coronary syndrome is high.

As shown in the Examples mentioned below, the plasma sAPP770 level/serum sAPP770 level ratio of acute myocardial infarction patients was significantly high as compared to that of healthy human. That is, the pathology of acute coronary syndrome can be determined by measuring the plasma and serum sAPP770 levels of the test subject and relating the plasma sAPP770 level/serum sAPP770 level ratio to the pathology of acute coronary syndrome. For example, when the plasma sAPP770 level/serum sAPP770 level ratio of a test subject is significantly higher than that of a healthy human, the test subject can be determined to have acute coronary syndrome (e.g., acute myocardial infarction).

Also, the therapeutic effect in patients undergoing treatments of acute coronary syndrome can be evaluated (i.e., determination of whether the prognosis of acute coronary syndrome is good or bad) using the method of the present invention. The therapeutic effect can be known by collecting plasma and/or serum from such patients before treatment, during treatment and/or after treatment, measuring the plasma and/or serum sAPP770 level(s), and examining changes thereof. For example, when the sAPP770 level of the plasma collected later is lower than that of the plasma collected earlier, the treatment can be evaluated as effective. When the sAPP770 level of the serum collected later is higher than that of the serum collected earlier, the prognosis can be evaluated as good. Also, when the ratio of the plasma sAPP770 level/serum sAPP770 level of the plasma and serum collected later is lower than that of the plasma and serum collected earlier, the prognosis can be evaluated as good.

When the plasma sAPP770 level/serum sAPP770 level ratio is used for relating to the pathology of acute coronary syndrome or onset risk, the cutoff value may be set in advance and the data of the test subject may be compared to the cutoff value. For example, when the plasma sAPP770 level/serum sAPP770 level ratio of the test subject is not less than the cutoff value, the data of the test subject is related to the pathology of acute coronary syndrome or onset risk, and whether the test subject suffers from acute coronary syndrome (e.g., acute myocardial infarction), or has a risk of developing acute coronary syndrome can be determined.

The "cutoff value" is a value that can satisfy both high diagnostic sensitivity (true positive rate) and high diagnostic specificity (true negative rate) when a disease is diagnosed using the value as a standard. For example, a value showing a high positive rate in an individual who developed acute coronary syndrome and a high negative rate in an individual who has not developed acute coronary syndrome can be set as a cutoff value.

The calculated method of the cutoff value is well known in the art. For example, a plasma sAPP770 level/serum sAPP770 level ratio is calculated in an individual who developed acute coronary syndrome and an individual who has not developed acute coronary syndrome, the diagnostic sensitivity and diagnostic specificity of the calculated values are determined, and an ROC (Receiver Operating Characteristic) curve is drawn based on these values and using a commercially available analysis soft. Then, a value showing diagnostic sensitivity and diagnostic specificity as close to 100% as possible is determined, and used as a cutoff value. It is also preferable to use, for example, "mean+2 standard deviation" of plasma sAPP770 level/serum sAPP770 level ratio of many healthy humans as the cutoff value. Using such value, the onset of acute coronary syndrome can be determined with good sensitivity and good specificity.

Among the above-mentioned embodiments of the present invention, when the pathology of acute coronary syndrome or onset risk is determined by calculating the plasma sAPP770 level/serum sAPP770 level ratio, the determination can be made with high reliability. Such embodiment is useful in any situation requiring determination of the pathology of acute coronary syndrome or onset risk. When the pathology of acute coronary syndrome or onset risk is determined by measuring the plasma sAPP770 level or serum sAPP770 level, the measurement of only one kind of sample is sufficient, which enables rapid and convenient determination at a low cost. Such embodiment is particularly useful for testing in a clinical setting requiring rapid processing, and combined use with the below-mentioned other diagnosis method. Therefore, which of plasma sAPP770 level, serum sAPP770 level, and plasma sAPP770 level/serum sAPP770 level ratio is to be used for the determination of the pathology of acute coronary syndrome or onset risk can be appropriately decided according to the situation requiring the determination.

The present invention can be used in combination with a known diagnosis method of acute coronary syndrome. Examples of the known diagnosis method include, but are not limited to, noninvasive examination (chest X-ray examination, electrocardiogram, echocardiography (ultrasonography of the heart), nuclear medicine study etc.), biochemical examination of blood (measurement of creatinine kinase, troponin T, troponin I, H-FABP, AST, CPK, LDH and the like, etc.), and invasive examination (coronary angiography, left ventriculography, CT scan etc.). In conventional invasive examination, even when stenosis of blood vessels is found, the stability of the plaque in the blood vessel cannot be determined, and the severity of acute coronary syndrome cannot be determined easily. However, a combined use with the test method of the present invention enables diagnosis with higher accuracy.

### 2. Diagnostic agent for determination of pathology or onset risk of acute coronary syndrome

The present invention further provides a diagnostic agent comprising sAPP770-specific antibody for determining the pathology or onset risk of acute coronary syndrome based on the plasma and/or serum sAPP770 level(s) of a test subject. Using the diagnostic agent of the present invention, the above-mentioned test method of the present invention can be performed with ease, and the pathology or onset risk of acute coronary syndrome can be determined rapidly and highly accurately.

The sAPP770-specific antibody contained in the diagnostic agent of the present invention is the same as the antibody described in "1. Test method of plasma and/or serum of test subject for determination of pathology or onset risk of acute coronary syndrome".

In one embodiment, the diagnostic agent of the present invention may further contain an antibody that recognizes sAPP different from the aforementioned sAPP770-specific antibody. The antibody that recognizes sAPP is the same as the antibody described in "1. Test method of plasma and/or serum of test subject for determination of pathology or onset risk of acute coronary syndrome".

The diagnostic agent of the present invention may consist only of the above-mentioned antibody(antibodies) alone or may contain a pharmaceutically acceptable carrier. When the diagnostic agent of the present invention is prepared as a liquid, the pharmaceutically acceptable carrier may contain various carriers conventionally used as preparation materials, for example, diluent, solvent, solubilizing agent, suspending agent, isotonicity agent, buffering agent and the like. Moreover, to prevent aggregation of antibody, a surfactant such as Tween 20 (registered trade mark) and the like are preferably added. The mixing ratio thereof can be appropriately determined by those of ordinary skill in the art.

The pathology or onset risk of acute coronary syndrome can be determined with ease by examining the plasma and/or serum of a test subject according to the aforementioned test method of the present invention and using the diagnostic agent of the present invention.

### 3. Diagnosis kit for determining pathology or onset risk of acute coronary syndrome

The present invention provides a kit for diagnosis, comprising sAPP770-specific antibody for determining the pathology or onset risk of acute coronary syndrome based on the plasma and/or serum sAPP770 level(s) of a test subject. The sAPP770-specific antibody contained in the kit for diagnosis of the present invention is the same as the antibody described in "1. Test method of plasma and/or serum of test subject for determination of pathology or onset risk of acute coronary syndrome".

The kit for diagnosis of the present invention may further contain an antibody that recognizes sAPP different from the aforementioned sAPP770-specific antibody. The "antibody that recognizes sAPP" is the same as the antibody described in "2. Diagnostic agent for determination of pathology or onset risk of acute coronary syndrome".

The diagnosis kit of the present invention may contain, besides the above-mentioned antibodies, other antibodies, reagents and the like. These antibody or reagent or the like may be combined with the above-mentioned antibodies in advance, or may be contained in separate containers. Examples of the antibody or reagent or the like include the secondary antibody, substrate, labeling substance (e.g., fluorescence dye, enzyme), solid phase, reaction container, as well as treatment liquid, buffer for diluting antibody, positive control (e.g., recombinant sAPP770), negative control, instruction describing protocol and the like. These factors can also be mixed in advance where necessary.

In the diagnosis kit of the present invention, the antibody may be solid phased in advance, and may be labeled in advance. The solid phase usable for the diagnosis kit of the present invention is not particularly limited and, for example, polymers such as polystyrene and the like, and insoluble carriers such as glass beads, magnetic particles, microplate, immunochromatography filter paper, glass filter and the like. Preferred is a microplate used for the sandwich ELISA method.

While the form of the diagnosis kit of the present invention is not particularly limited, an integrated diagnosis kit, wherein the components of the diagnosis kit of the present invention are integrated, can be provided to achieve convenient diagnosis. Examples of the form of the integrated diagnosis kit include a cassette type using an immunochromatography method.

Using the diagnosis kit of the present invention, the determination of the pathology or onset risk of acute coronary syndrome can be performed more conveniently.

### Examples

The present invention is explained in detail in the following by referring to the following Examples, which are not to be construed as limitative. The reagent, apparatus and materials used in the present invention are commercially available unless otherwise indicated.

### Material and method

### (Subjects)

The clinical study was approved by the ethics committee of RIKEN, Tohoku University and Fukushia Medical University.

As the research target group including ACS patients, effort angina pectoris and unstable angina pectoris patients, patients admitted to Fukushia Medical University Hospital were registered. Peripheral blood samples were collected when they visited the hospital. AMI patients were diagnosed by coronary angiography to observe coronary obstruction, and treated by percutaneous transluminal coronary angioplasty. Peripheral blood was collected from AMI patients when they were admitted.

### (Materials)

The sources of materials used in the Examples are as follows: tissue culture medium and reagent (including DMEM) from Invitrogen; protein molecular weight standard from Bio-Rad; recombinant human IL-1β, TNFα and IL-6 from R&D Systems; TAPI0 from Peptide Institute Inc.; collagen from Nycomed; all other chemical products from Sigma or Wako Chemicals. Anti-APP(C) antibody recognizes C-terminal part of APP. Anti-OX2 antibody was prepared using synthetic oligopeptide TTQEPLARDPVKL conjugated with keyhole limpet hemocyanin as an antigen. Commercially available antibodies used in the Examples were mouse monoclonal anti-APP 22C11 (Chemicon) antibody and anti-Kunitz-type protease inhibitory (KPI) region (Chemicon) antibody. SD rats were purchased from CLEA Japan, Inc.

### (Expression plasmid and cell culture)

Human APP695-pcDNA3.1, APP751-pcDNA3.1 and APP770-pcDNA3.1 used were those constructed in the previous study (J Biol Chem 285(51):40097-40103 (2010)). Human BMEC (Applied Cell Biology Research Institute) was cultured in CS-C complete medium added with 10% FBS, and used within 4 passages.

### (Preparation of human PRP and platelet)

On the day of experiment, blood (16 ml) was collected from healthy human by using a vacuum blood collection tube (NIPRO CORPORATION) containing 3.8% sodium citrate. PRP (2.3x10⁸-2.7x10⁸ platelets/ml) was recovered by centrifugation at 200 x g for 20 min. Platelets were recovered from PRP by centrifugation at 900 x g for 10 min in the presence of 4 mM citrate. Platelet pellets were resuspended in modified HEPES-Tyrode buffer (134 mM NaCl, 12 mM NaHCO₃, 2.9 mM KC1, 0.34 mM NaH₂PO₄, 1 mM CaCl₂, 5 mM Hepes, 5 mM glucose, pH 7.4) at a density of 2.5x10⁸ platelets/ml. PRP or an aliquot of the platelet suspension (200 µl) was used for platelet aggregation assay. The platelet aggregation assay was performed using TPA-4C aggregometer (Tokyo Photo Electric Co., Ltd.) by constantly stirring the suspension at 1,000 rpm in a silicon glass cuvette at 37°C. Collagen (final concentration 3 µg/ml) was added to start platelet aggregation. PRP or platelet suspension was centrifuged at each time point, and platelet pellets were solubilized with T-PER buffer (Thermo Fisher Scientific Inc., 200 µl) containing complete protease inhibitor cocktail (Roche). The obtained cell lysate was evaluated by ELISA and Western blot analysis.

### (Quantification of sAPP using ELISA system)

Using human sAPP total assay kit (IBL-Japan), sAPP695, sAPP751 and sAPP770 were detected according to the established protocol (http://www.ibl-japan.co.jp). The APP770 ELISA system was established as previously reported (Anal Chim Acta 631(1): 116-120 (2009)). Briefly explained, a 96 well plate was coated with an anti-OX2 antibody, and a horseradish peroxidase-conjugated anti-APP R101A4 antibody was used as a detection antibody. Human CSF (1:16 diluted with dilution buffer contained in kit), plasma and serum (1:75 diluted with dilution buffer contained in kit) were evaluated by APP total ELISA and APP770 ELISA.

### (Detection of APP)

COS cells expressing human APP695, APP751 or APP770, or platelet lysate was solubilized with T-PER buffer containing complete protease inhibitor cocktail. sAPP in the platelet release product was pulled down with heparin-agarose (Thermo Fisher Scientific Inc.). COS cell lysate (containing 5 µg protein) and platelet-derived sample (corresponding to about 5x10⁶ platelets) were subjected to SDS-PAGE (5-20% gradient gel), and transferred on a nitrocellulose membrane. In Western blot analysis, the membrane was incubated with anti-APP 22C11 antibody (1:1,000 diluted), anti-OX2 antibody (1:100 diluted), anti-APP(C) antibody (1:1,000 diluted) or anti-KPI antibody (1:1,000 diluted). As the secondary antibody, horseradish peroxidase-conjugated donkey anti-goat IgG antibody (Jackson ImmunoResearch Laboratories), anti-mouse IgG antibody and anti-rabbit IgG antibody (GE Healthcare) were used (1:1,000 diluted). For detection of the bound antibody, ECL Prime Blocking Agent and ECL Advanced Chemiluminescent Substrate (GE Healthcare) were used. Luminoimage Analyzer LAS-1000 PLUS (Fujifilm Corporation) was used to quantify the detected signals.

### (Measurement of sAPPα level)

Rat sAPPα was measured using rat/mouse sAPPα ELISA assay kit of Japan Immuno-Biological Laboratories Co., Ltd. Serum and plasma (about 0.5 ml) were collected over time for 0, 1 and 2 hr from myocardial infarction model rat with ligated coronary artery and control rat with only a laparotomy, diluted 4-fold with a buffer contained in the kit, and measured using the ELISA kit.

### (Measurement of cardiac muscle troponin I (cTn-I) level)

ELISA kit of Life Diagnostics, Inc. was used for cardiac muscle troponin I in rat serum. Sera of myocardial infarction and control rats obtained by the aforementioned method were diluted 5-fold with the contained dilution buffer, and measured by ELISA.

### (Measurement of creatine kinase (CK-MM) level)

ELISA kit of Life Diagnostics, Inc. was used for CK-MM in the rat serum. Sera of myocardial infarction and control rats obtained by the aforementioned method were diluted 15-fold with the contained dilution buffer, and measured by ELISA.

### Examples

The present inventors have developed a sandwich ELISA system that specifically detects APP770, and does not detect APP695 and APP751 (WO2012/015050). In this ELISA system, an antibody to OX2 domain which is a domain inherent to APP770 is used. As shown in Fig. 1B, the anti-N terminal APP antibody (22C11) detected APP695, APP751 and APP770, whereas the anti-OX2 antibody specifically detected APP770 alone. When the anti-N terminal APP antibody and anti-OX2 antibody were used, the APP770 sandwich ELISA system has a linear range of 50 pg/ml - 9 ng/ml.

In this Example, conventional APP total ELISA was also used for comparison. APP total ELISA detected sAPP695, sAPP751 and sAPP770 secreted from COS cells that transiently over-express APP695, APP751 and APP770, respectively. In contrast, APP770 ELISA detected only sAPP770 (Fig. 1C). Furthermore, the level of endothelial sAPP770 secreted from human brain microvascular endothelial cell (BMEC) was also detected by the ELISA (Fig. 1D). IL-1β is known to promote sAPP secretion from endothelial cell (Proc Natl Acad Sci USA 89(21): 10075-10078 (1992)) and neuron (J Neurochem 104(5): 1387-1393 (2008)). In fact, the level of sAPP770 secreted from BMEC significantly increased only slightly by the addition of IL-1β and TNFα, and did not increase by the addition of IL-6 (Fig. 1D). Since selective TACE inhibitor TAPI-0 partially inhibited secretion of sAPP770 from BMEC (Fig. 1E), TACE is involved at least partially in the production of sAPP770α in the endothelial cells, as in the case of sAPP695 production (Journal of Neuroscience 28(46): 12052-12061 (2008)). Then, APP770 ELISA was applied to human serum sample and human plasma sample. While the plasma of healthy individual contained sAPP770 at about 99.7±13 ng/ml, the serum of healthy individual unexpectedly contained sAPP770 at a higher level (about 376±19 ng/ml), which was almost 4-fold the plasma sAPP770 level (Fig. 1F).

Since sAPP having a Kunitz protease inhibitory domain is found in a large amount as a platelet α granule protein (called protease nexin-2 (PN2)), and release of PN2/APP from platelet due to the degranulation has been reported heretofore (Science 248(4956):745-748 (1990), J Biol Chem 265(26):15977-15983 (1990)), it was assumed that the significantly high serum level of sAPP770 is mainly caused by platelet. To examine whether platelets express APP770, and whether activation of platelets results in the release of APP770, platelet-rich plasma (PRP) was first isolated, collagen was added to stimulate platelets. After centrifugation, the APP770 level of the supernatant and platelet precipitate was measured. One minute after collagen stimulation, the level of sAPP770 in the PRP supernatant increased rapidly (Fig. 2A). Corresponding thereto, the level of sAPP770 in the platelet precipitate decreased remarkably (Fig. 2B). When the platelet was isolated and stimulated with collagen, sAPP770 was released as in the case of PRP (Fig. 2C). Therefrom it was concluded that platelet expresses APP770 and releases APP770 by activation. Western blot analyses of resting platelet and activated platelet using a series of anti-APP antibodies (Fig. 2D) showed that sAPP (about 120 kDa, gray arrow head) detected by 22C11 but not detected by anti-APP(C) antibody is already present in the resting platelet (Fig. 2E). Since the sAPP signal was also detected by anti-OX2 antibody and anti-KPI antibody, the actual presence of sAPP770 in the platelet was confirmed. In agreement with the decrease in sAPP770 in the platelet due to activation, sAPP770 in the platelet release product (platelet releasate) remarkably increased. In addition to sAPP770, full-length APP770 (about 140 kDa, black arrow head) (detected by both anti-APP(C) antibody and anti-OX2 antibody) were also present at a far lower level. The results show that cleavage of APP770 does not contribute much to the release of APP770 by platelet activation.

Then, to clarify the proportion of sAPP770 in the total sAPP in in vivo samples, the sAPP total level and sAPP770 level of a healthy human-derived human serum sample and a human brain spinal cord fluid (CSF) sample were measured. The proportion of sAPP770 in CSF was about 7.4%, which was remarkably lower than that of serum sAPP770 (about 46%) (Table 1). This may be because the blood-brain barrier prevents flow of the serum sAPP770 into the brain. Therefore, sAPP total in CSF is mostly derived from neuron sAPP695, whereas serum sAPP total is considered to be mostly derived from sAPP770. Due to the technical limitation that the standard sample is different between APP770 ELISA and APP total ELISA, direct comparison of the sAPP770 level and sAPP total level may not be very accurate.

**Table 1**

| | | sAPP770 | sAPP total |
|---|---|---|---|
| serum | (ng/ml) | 413±69.5 | 894±89.0 |
| (n=20) | rate (%) | 46.1 | 100 |
| CSF | (ng/ml) | 54.9±7.98 | 742±88.3 |
| (n=12) | rate (%) | 7.4 | 100 |

Since it was clarified that platelet activation releases APP770, serum and plasma sAPP770 in acute coronary syndrome (ACS) patients was analyzed. It is well known that the initial endothelial damage and subsequent platelet activation are closely associated with the pathological cascade of ACS (N Engl J Med 326(4):242-250 (1992)). First, APP770 in the plasma samples collected from the artery of stable angina pectoris (angina pectoris) (stable AP) patients (effort angina pectoris patients), unstable AP patients and AMI patients during a catheter treatment of the heart was analyzed. The plasma APP770 level became higher in the order of stable AP case, unstable AP case, and AMI case (Fig. 4). This suggests that the grade of the endothelial damage and platelet activation can be monitored by plasma APP770 level. Next, to evaluate the practicality, sAPP770 in plasma and serum samples derived from the peripheral blood of normal cases and normal cases and AMI patients was analyzed. As compared to the normal cases, AMI patients showed a significantly high level of plasma APP770 (Fig. 3A) and an unexpectedly low level of serum APP770 (Fig. 3B). As a result, the ratio of plasma sAPP770 to serum sAPP770 in AMI patients was about 1.06±0.16, which was remarkably higher than in the normal cases (about 0.26±0.03) (Fig. 3C). The cutoff value of plasma sAPP770/serum sAPP770 ratio was set to 0.48 which corresponds to mean+2SD of normal cases. As a result, 94.4% of the normal cases were lower than the cutoff value, and 100% of the AMI patients were higher than the cutoff value. These results show that the sensitivity and specificity of the plasma sAPP770/serum sAPP770 ratio are 100% and 94%, respectively, thus emphasizing the usefulness of the ELISA for the diagnosis and management of AMI.

Furthermore, the present inventors produced myocardial infarction (MI) model rats, and verified the usefulness of sAPP770 as an early diagnosis marker of myocardial infarction.

The MI model rats were produced by laparotomy of SD rats to ligate coronary artery. As in the case of MI model rats, rat with laparotomy but without ligation of coronary artery was used as a control. Plasma and serum were collected before ligation (0 h) and 1 and 2h after ligation, and plasma rat sAPPα level, and serum cardiac muscle troponin-I(cardiac Troponin-I (cTn-I)) level and creatine kinase (CK-MM) level were measured.

Since sAPP770 measurement system of rat was under development, rat/mouse sAPPα ELISA was used here. Of sAPP, sAPP770 is the major component of blood, and the amount of sAPPα is overwhelming higher than sAPPβ, and therefore, sAPP770 level was considered to be comprehensible by measuring the sAPPα level.

As a result, sAPPα of MI model rat increased to not less than 4-fold at 1 h after ligation as compared to the control (Fig. 5A), whereas myocardial infarction markers cTn-I and CK-MM increased to not more than 2-fold at 1 h after ligation (Fig. 5B, C). Therefrom it is clear that sAPP770 increases before deviation enzyme marker from the cardiac muscle, and the usefulness thereof as an early diagnosis marker of myocardial infarction was shown.

### Industrial Applicability

According to the present invention, the pathology of acute coronary syndrome can be determined rapidly and highly accurately based on the plasma and/or serum sAPP770 level(s), whereby rapid diagnoses and optimal treatments can be provided in clinical practice.

This application is based on a patent application No. 2012-129272 filed in Japan (filing date: June 6, 2012), the contents of which are incorporated in full herein.

## Claims

1. A method of testing plasma and/or serum of a test subject for determining the pathology or onset risk of acute coronary syndrome, comprising
(1) a step of measuring a soluble amyloid β precursor protein (sAPP) 770 level of plasma and/or serum of a test subject; and
(2) a step of relating the plasma and/or serum sAPP770 level(s) measured in (1) to the pathology or onset risk of acute coronary syndrome.

2. The method according to claim 1, wherein the step (1) comprises measuring the plasma sAPP770 level of the test subject and the step (2) comprises relating the plasma sAPP770 level to the pathology or onset risk of acute coronary syndrome.

3. The method according to claim 1, wherein the step (1) comprises measuring the plasma and serum sAPP770 levels of the test subject and the step (2) comprises relating the ratio of plasma sAPP770 level/serum sAPP770 level to the pathology or onset risk of acute coronary syndrome.

4. The method according to claim 1, wherein the step (1) comprises measuring the serum sAPP770 level of the test subject, and the step (2) comprises relating the serum sAPP770 level to the pathology or onset risk of acute coronary syndrome.

5. The method according to any one of claims 1 to 4, wherein the step (2) comprises relating the plasma and/or serum sAPP770 level(s) measured in step (1) to the pathology of acute coronary syndrome.

6. The method according to claim 5, which is a test method of plasma and/or serum of a test subject for determining severity of acute coronary syndrome.

7. The method according to claim 5, which is a test method of plasma and/or serum of a test subject for distinguishing acute coronary syndrome from non-acute coronary syndrome.

8. The method according to claim 5, which is a test method of plasma and/or serum of a test subject for determining whether the prognosis of acute coronary syndrome is good or bad.

9. The method according to any one of claims 1 to 4, which is a test method of plasma and/or serum of a test subject for determining the onset risk of acute coronary syndrome, wherein the step (2) comprises relating the plasma and/or serum sAPP770 level(s) measured in the step (1) to the onset risk of acute coronary syndrome.

10. The method according to any one of claims 1 to 9, wherein the sAPP770 level is detected using an sAPP770-specific antibody.

11. The method according to claim 10, wherein the sAPP770-specific antibody recognizes an OX2 domain.

12. The method according to any one of claims 1 to 11, wherein the sAPP770 level is measured by sandwich ELISA.

13. A diagnostic agent comprising an sAPP770-specific antibody for determining the pathology or onset risk of acute coronary syndrome based on the plasma and/or serum sAPP770 level(s) of a test subject.

14. The agent according to claim 13, wherein the sAPP770-specific antibody recognizes an OX2 domain.

15. The agent according to claim 13 or 14, further comprising an antibody recognizing sAPP, which is different from the aforementioned sAPP770-specific antibody.

16. A diagnosis kit comprising an sAPP770-specific antibody for determining the pathology or onset risk of acute coronary syndrome based on the plasma and/or serum sAPP770 level(s) of a test subject.

17. The kit according to claim 16, wherein the sAPP770-specific antibody recognizes an OX2 domain.

18. The kit according to claim 16 or 17, further comprising an antibody recognizing sAPP, which is different from the aforementioned sAPP770-specific antibody.
